Europäisches Patentamt

European Patent Office

Office européen des brevets

(19)

(11) Veröffentlichungsnummer: **0 242 778**
**B1**

(12) **EUROPÄISCHE PATENTSCHRIFT**

(45) Veröffentlichungstag der Patentschrift:
13.09.89

(21) Anmeldenummer: 87105531.5

(22) Anmeldetag: 14.04.87

(51) Int. Cl.⁴: **C 07 B 57/00,** C 07 C 69/743,
C 07 C 67/54

(54) Trennung von Diastereomeren.

(30) Priorität: 25.04.86 DE 3613975

(43) Veröffentlichungstag der Anmeldung:
28.10.87 Patentblatt 87/44

(45) Bekanntmachung des Hinweises auf die Patenterteilung:
13.09.89 Patentblatt 89/37

(84) Benannte Vertragsstaaten:
CH DE FR GB IT LI

(56) Entgegenhaltungen:
FR-A-2 176 621
US-A-3 491 152

ULMANNS ENCYCLOPÄDIE DER TECHNISCHEN
CHEMIE, 4.Auflage, Band 2, Seiten 511/12, Verlag
Chemie, Weinheim, Bergstrasse

(73) Patentinhaber: BAYER AG, D-5090 Leverkusen 1
Bayerwerk (DE)

(72) Erfinder: Arlt, Dieter, Prof. Dr., Rybniker Strasse
2, D-5000 Köln 80 (DE)
Erfinder: Schwartz, Ulrich, Dr., Wiesdorfer Platz
10, D-5090 Leverkusen 1 (DE)
Erfinder: Brandt, Hans- Walter, Dr., Zum
Hahnenberg 64, D-5068 Odenthal (DE)
Erfinder: Arlt, Wolfgang, Dr., Am Gartenfeld 48,
D-5068 Odenthal (DE)
Erfinder: Nickel, Andreas, Dipl.- Ing., Lütge
Varney 10, D-5802 Wetter 4 (DE)

**Beschreibung**

Die vorliegende Erfindung betrifft die Trennung von diastereomeren Verbindungen zum Erhalt sterisch einheitlicher Stoffe, die z. B. als biologische Wirkstoffe oder als Zwischenprodukte für die Herstellung biologischer Wirkstoffe eingesetzt werden.

Die biologischen Eigenschaften chemischer Verbindungen sind häufig mit einer bestimmten sterischen Struktur eines Wirkstoffmoleküls verknüpft. Daher ist man bestrebt, sterisch einheitliche und biologisch hochwirksame Verbindungen herzustellen oder aus Gemischen der verschiedenen Stereoisomeren zu isolieren. Diese Aufgabe ist in Folge der geringen (physikalischen und/oder chemischen) Eigenschaftsunterschiede von Stereoisomeren nur aufwendig zu lösen. Bisher bekannte Verfahren zur Trennung von Diastereomeren sind die fraktionierte Kristallisation und die fraktionierte Destillation. Diastereomere sind alle Stereoisomere, die nicht Enantiomere sind, sich also nicht wie Bild und Spiegelbild verhalten. Die Trennung von Diastereomeren durch fraktionierte Kristallisation kann natur gemäß nur auf kristallisationsfähige Substanzen angewendet werden und führt häufig zu unbefriedigenden Ausbeuten. Die Trennung von Diastereomeren durch Destillation ist auch auf Flüssigkeiten anwendbar, versagt aber häufig völlig. In den wenigen praktisch durchführbaren Fällen ist ein außerordentlich großer apparativer Aufwand wegen der erforderlichen großen Trennstufenzahl notwendig. Damit verbunden ist ein hoher kostspieliger Energieaufwand für die Destillation. Es bestand also ein dringendes Bedürfnis, neue und besser praktikable Verfahren zur Trennung von Diastereomeren zu finden, welche die Nachteile o.g. Trennverfahren nicht aufweisen.

In der Publikation FR-A-2 176 261 wird in erster Linie die Trennung optischer Isomere mit Hilfe der Extraktivdestillation beschrieben, wobei im Gegensatz zum anmeldungsgemäßen Verfahren ausschließlich optisch aktive Hilfsstoffe zugesetzt werden. Das Wesentliche ist also bei FR-A-2 176 261 eine sterische Interaktion zur Hervorrufung des gewünschten Trenneffekts. Siehe dazu insbesondere Seite 2, Zeilen 21 bis 23 von FR-A-2 176 621.

Die Publikation Ullmann's Enzyklopädie der Technischen Chemie, Band 2, Seite 511f. bezieht sich nicht auf die Trennung von Diastereomeren. Als Beispiel wird in der letztgenannten Veröffentlichung die extraktive Rektifikation von Benzol/Cyclohexan mit Anilin als Extraktionsmittel angegeben.

Es war überraschend, daß durch das erfindungsgemäße Verfahren, wie durch die Beispiele der vorliegenden Anmeldung belegt, eine derartig gute Trennung der einzelnen Stereoisomeren mit einem relativ geringfügigen Arbeitsaufwand möglich war.

Es wurde nun gefunden, daß man Diastereomere der Reihe cis/trans Permethrinsäuremethylester(3-(2,2-Dichlorvinyl)-2,2-dimethyl-cyclopropancarbonsäure-methylester) oder cis/trans Permethrinsäureethylester (3-(2,2-Dichlorvinyl-2,2-dimethyl-cyclopropancarbonsäureethylester) oder cis/trans Permethrinsäurechlorid (3-(2,2-Dichlorvinyl)-2,2-dimethyl-cyclopropancarbonsäurechlorid) oder cis/trans 2,2-Dimethyl-cyclopropan-3-carbonsäuremethylester-1-aldehyd oder Menthol/Isomenthol mit sehr gutem Erfolg dadurch auftrennen kann, daß man sie mit Hilfe der Extraktivdestillation in einer Fraktionierkolonne bei Drücken von ca. 1 bar bis ca. 1 mbar unter Zusatz eines polaren Hilfsstoffes mit einem Siedebereich von 50°C bis 310°C der Reihe Amine, Ether, Amide, Ketone, Alkohole, Nitrile, Heterocyclen, Sulfoxide, Sulfone durchführt, wobei die Auswahl des polaren Hilfsstoffes in einem Vorversuch mit Hilfe der Dampfraumanalyse erfolgt.

Überraschenderweise gelingt es mit Hilfe des erfindungsgemäßen Verfahrens, mit wesentlich geringerem Apparate- und Energieaufwand die genannten Diastereomere in guter Raum-Zeit-Ausbeute zu trennen. Hierbei erfolgt die Auftrennung in Fraktionierkolonnen bei Drücken von $10^{-3}$ bar bis 1 bar.

Als geeignete polare Hilfsstoffe kommen beim erfindungsgemäßen Verfahren Amine, Ether, Amide, Ketone, Alkohole, Nitrile, Heterocyclen, Sulfoxide, Sulfone in Frage.

Als geeignete Stoffe seien beispielhaft genannt: Glycerin, Diethanolamin, Diphenylether, Acetamid, N-Methylacetamid, N-Methylformamid, Aceton, Furfurol, Acetonitril, Dimethylformamid, Dimethylacetamid, N-Methylpyrrolidon, N-Formylmorpholin, Benzylalkohol, Phenol, Dimethylsulfoxid, Sulfolan, Glutarsäuredinitril, Bernsteinsäuredinitril, Bernsteinsäureamid, 1-Methyloxo-phospholin und N-Methylcaprolactam. Insbesondere wird erfindungsgemäß in vielen Fällen vorteilhaft Sulfolan, 4-Methylmorpholin, Methylformamid, 2-Pyrrolidon, N-Methylacetamid, Glutarsäuredinitril, Bernsteinsäureredinitril, Bernsteinsäureamid und 1-Methyl-oxo-phospholin als Hilfsstoff eingesetzt.

Die zuzusetzenden Hilfsstoffe ermöglichen oder verbessern die Trennung eines vorgegebenen Diastereomeren-Gemisches durch Destillation, indem sie die Wechselwirkungen der im Gemisch vorhandenen Moleküle derart verändern, daß eine oder mehrere Molekülsorten durch die Anwesenheit des Hilfsstoffes eine Änderung der Wechselwirkungsenergie erfahren, die dazu führt, daß eine oder mehrere Molekülsorten nun flüchtiger werden als andere.

Die Auswahl der für die Trennung einzusetzenden Hilfsstoffe erfolgt über die Bestimmungsmethode der Dampfraumanalyse. Bei dieser Methode wird das zu trennende Substanzgemisch (Diastereomerengemisch) in ein thermostatisiertes Gefäß eingefüllt und die Zusammensetzung der entstehenden Dampfphase durch Gaschromatographie analytisch bestimmt.

Aus den Einwaagen der Flüssigkeit, vermindert um die entstandene Dampfmenge, und der analytisch bestimmten Dampfkonzentration läßt sich der Trennfaktor genau bestimmen. Zur Feststellung des Einflusses der erfindungsgemäßen Hilfsstoffe werden diese zusammen mit dem zu trennenden Substanzgemisch in den thermostatisierten Gefäßen vorgelegt. Die Bestimmung des Trennfaktors erfolgt wie bereits beschrieben. Die Vorauswahl der erfindungsgemäß gefundenen Hilfsstoffe richtet sich nach deren Siedepunkt, der höher sein sollte als derjenige des zu trennenden Substanzgemisches, der Mischbarkeit mit dem zu trennenden Substanzgemisch sowie nach seiner Destillierbarkeit, ohne daß unerwünschte Zersetzung auftritt.

Ein Maß für die Trennungsmöglichkeit eines Diastereomeren-Gemisches durch Destillation ist der Trennfaktor.

Ein Trennfaktor von Eins bedeutet, daß ein Gemisch destillativ nicht trennbar ist, Werte größer als Eins bedeuten steigende Trennbarkeit.

Die Bestimmung des Trennfaktors erfolgt über die vorstehend beschriebene Dampfraumanalyse. Weitere Charakteristika für die destillative Trennung sind die Trennstufenzahl und das Rücklaufverhältnis, die im folgenden kurz definiert werden sollen.

Trennstufenzahl: Die Trennstufenzahl ist die Anzahl der Gegenstrom-Austauscheinheiten in einer Destillationskolonne. Diese entsprechen den Böden einer Bodenkolonne bzw. einer bestimmten Packungshöhe bei Kolonnen mit Füllkörpern.

Rücklaufverhältnis: Das Rücklaufverhältnis gibt die Aufteilung des am Kondensator anfallenden Stoffstromes an. Es ist der Quotient aus dem in die Kolonne zurückfließenden Strom und dem Entnahmestrom. Kleine Werte des Rücklaufverhältnisses bedeuten geringen Energieverbrauch, große Werte großen Energieverbrauch pro kg erzeugtem Destillat.

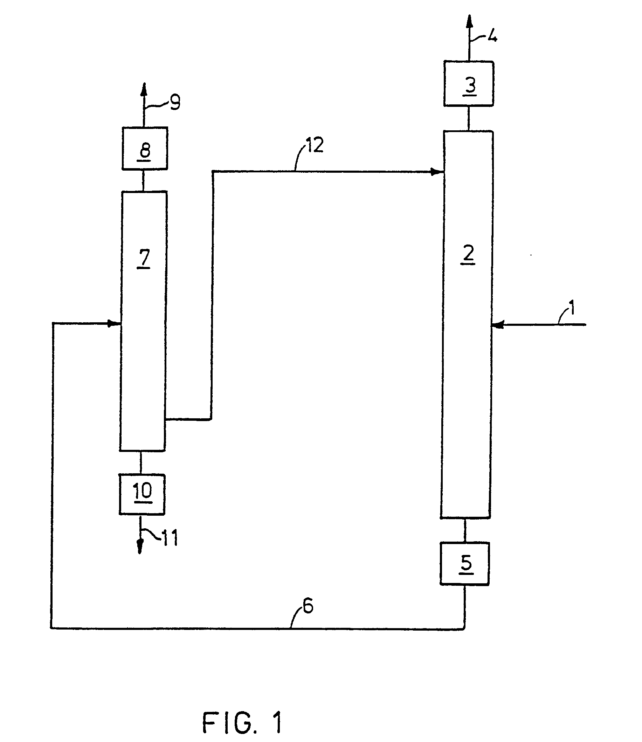

Die technische Durchführung der Extraktivdestillation ist an sich bekannt, für das erfindungsgemäße Verfahren kommt vorzugsweise die Ausführung nach Fig. 1 in Betracht.

2 ist die Extraktivdestillationskolonne mit ihrem Kondensator 3 und dem Verdampfer 5. In diese Kolonne fließt das erfindungsgemäß zu trennende Diastereomerengemisch aus mindestens zwei Isomeren mit Strom 1. Strom 12 ist der beim erfindungsgemäßen Verfahren eingesetzte Hilfsstoff, der die Trennung des mit 1 zulaufenden Gemisches bewirkt. Strom 4 sind eine oder mehrere vom Zulaufgemisch getrennte Isomeren. Die verbleibenden Isomeren fließen mit Strom 6 in die Aufarbeitungskolonne 7 mit ihrem Verdampfer 10 und dem Kondensator 8. Der oder die verbleibenden Isomeren werden mit Strom 9 vom Hilfsstoff befreit, aus der Kolonne entnommen. Der gereinigte Hilfsstoff fließt mit Strom 12 in die Extraktivdestillationskolonne 2 zurück. Durch thermische oder andere Zersetzung entstandene hochsiedende Produkte werden mit Strom 11 entnommen.

Die überraschende Überlegenheit des erfindungsgemäßen Verfahrens zur Trennung diastereomerer Verbindungen gegenüber einer destillativen Trennung durch fraktionierende Destillation ohne Hilfsstoff zeigen die folgenden Beispiele und Vergleichsbeispiele, wobei zu berücksichtigen ist, daß in vielen Fällen eine Trennung durch eine einfache fraktionierende Destillation mißlingt.

**Beispiel 1**

Trennung von cis/trans Permethrinsäuremethylester (3-(2,2-Dichlorvinyl)-2,2-dimethyl-cyclopropancarbonsäuremethylester).

Vorgabe: Gemisch enthaltend 60 % cis-Isomeres und 40 % trans-Isomeres
Ziel: 99-%-ige Isomerenreinheit in den Produktströmen 4 und 9 der Figur 1.

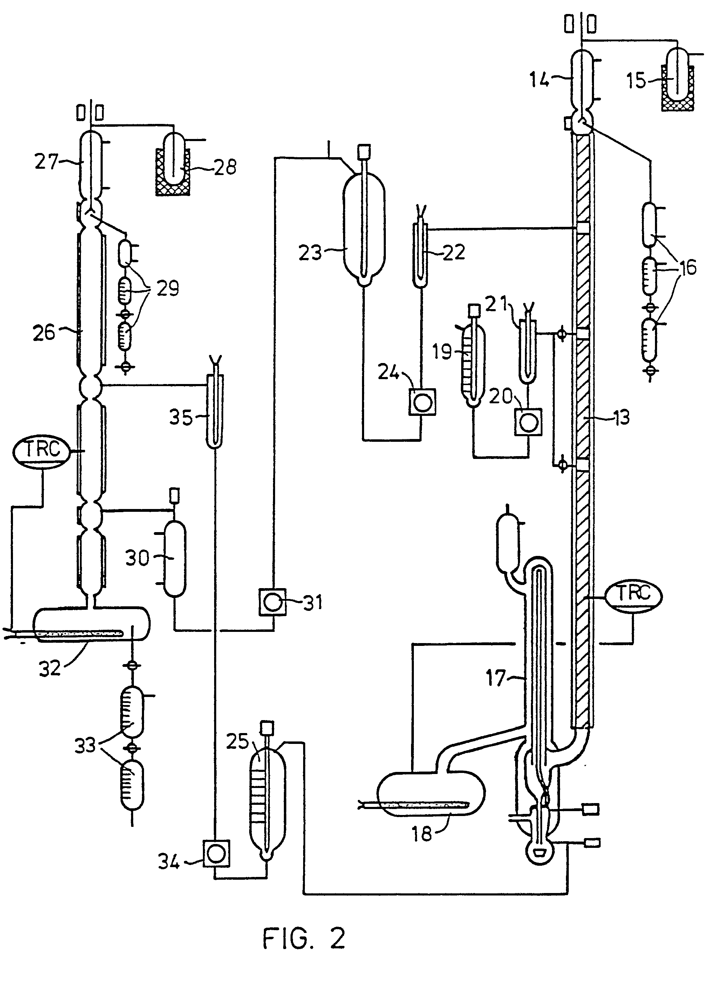

Die Trennung wurde nach dem Prinzip der Figur 1 durchgeführt. Die genaue Versuchsdurchführung ist aus der Figur 2 unter Angabe der stündlichen Stoffströme und der Versuchsbedingungen zu entnehmen.

In der Figur 2 bedeutet:

| | |
|---|---|
| 13 | Extraktivdestillationskolonne |
| 14 | Kondensator |
| 15 | Kühlfalle |
| 16 | Destillatvorlage |
| 17 | Verdampfer |
| 18 | Siedethermostat |
| 19 | Isomerengemischvorlage |
| 25 | Sumpfvorlage |
| 21/22 | Zulaufvorwärmer |
| 23 | Sulfolanvorlage |
| 20/24 | Zulaufpumpen |
| 26 | Aufarbeitungskolonne |
| 27 | Kondensator |
| 28 | Kühlfalle |
| 29 | Destillatvorlage |
| 30 | Seitenstromkondensator |
| 31 | Seitenstrompumpe |
| 32 | Verdampfer |

33 Sumpfvorlage
34 Zulaufpumpe
35 Zulaufvorwärmer

Als Hilfsstoff für die Trennung der beiden Diastereomeren wird hier Sulfolan (Tetramethylensulfon) eingesetzt.

Aufarbeitung von cis/trans Permethrinsäuremethylester (= PSME) mit Sulfolan: Betriebsbedingungen und stündliche Bilanz.

Extraktivdestillationskolonne (13):
13,5 m (Sulzer BX DN 70)
Temperatur im unteren Bereich von (13): 165° C,
Druck im unteren Bereich von (13): 50 mbar,
Temperatur im Mittelbereich von (13): 140° C,
Temperatur im Kondensator (14): 114° C,
Druck im Kondensator (14): 30 mbar
Menge Rücklauf zu Menge Entnahme (= R/E) im Kondensator (14) = 3 : 1.

In der Destillatvorlage (16) erhält man ein Gemisch aus 243,4 g cis PSME 0,5 g trans PSME und 5,0 g Sulfolan, wobei in die Isomerengemischvorlage (19) ein Gemisch bestehen aus 247 g cis PSME und 228 g trans PSME eingegeben wurde. In der Destillatvorlage (29) erhält man ein Gemisch aus 3,6 g cis PSME, 227,5 g trans PSME und 2,0 g Sulfolan. Aus der Sulfolanvorlage (23) werden 3400 g Sulfolan pro Stunde zugegeben.

Die Temperatur im Siedethermostat (18) beträgt 187° C, im Verdampfer (32) ca. 175° C, in der Lösungsmittelabtrennungskolonne (26) (4 x 4 Maschendrahtringe, DN 50, 2 x 2000 mm) 170° C und im Kondensator (27) 117° C.

Der Druck im Verdampfer (32) beträgt 45 mbar und im Kondensator (27) 30 mbar. Das Rücklaufverhältnis R/E ist im Kondensator (27) = 1,5.

In der Sumpfvorlage (33) sind keine hochsiedenden Substanzen angefallen. Vakuum wird in der Apparatur an folgenden Stellen angelegt:
Kühlfalle (15), Verdampfer (17), Kühlfalle (28).

TRC bedeutet in der Figur 2 Temperaturregister-Regelung.

| **Ergebnis:** | ohne Hilfsstoff | mit Hilfsstoff Sulfolan |
|---|---|---|
| Trennfaktor | 1,15 | 1,74 |
| Stufenzahl | 150 | 25 |
| Rücklaufverhältnis | 12 | 4 |

Das Beispiel zeigt also, daß die benötigte Trennstufenzahl von 150 auf 25 und das Rücklaufverhältnis von 12 auf 4 gesenkt werden konnte. Es gelang jeweils 99 % reines cis- bzw. trans-Isomeres zu isolieren.

**Beispiel 2**

Trennung von cis/trans Permethrinsäuremethylester

An Stelle von Sulfolan können gemäß der Anordnung von Beispiel 1 auch folgende Hilfsstoffe für die Trennung der beiden Diastereomeren eingesetzt werden:
4-Methylmorpholin, Methylformamid, 2-Pyrrolidon, N-Methylacetamid, 1-Methyl-oxo-phospholin.

**Beispiel 3**

Trennung von Menthol/iso-Menthol

Gemäß der Versuchsanordnung des Beispiels 1 und unter Verwendung von Bernsteinsäurediamid als Hilfsstoffs können Menthol/iso-Menthol erfolgreich getrennt werden.

| **Ergebnis:** | ohne Hilfsstoff | mit Bernsteinsäurediamid |
|---|---|---|
| Trennfaktor | 1,09 | 1,44 |
| Stufenzahl | 194 | 31 |
| Rücklaufverhältnis | 19 | 5 |

Bedingungen: 50/50 Zulauf, 1 % Leichtsieder im Sumpf, 10 % Schwersieder im Kopf

Für die Trennung Menthol-iso-Menthol können weiterhin beispielsweise folgende Hilfsstoffe eingesetzt werden:
Glutarsäuredinitril, Bernsteinsäuredinitril, Campher, Malonsäuredinitril.

## Patentansprüche

1. Verfahren zur Trennung von Diastereomeren der Reihe cis/trans Permethrinsäuremethylester (3-(2,2-Dichlor-vinyl)-2,2-dimethyl-cyclopropancarbonsäure-methylester) oder cis/trans Permethrinsäureethylester (3-(2,2-Dichlor-vinyl)-2,2-dimethyl-cyclopropancarbonsäureethylester) oder cis/trans Permethrinsäurechlorid (3-(2,2-Dichlor-vinyl)-2,2-dimethyl-cyclopropan-carbonsäurechlorid) oder cis/trans 2,2-Dimethyl-cyclopropan-3-carbonsäuremethylester-1-aldehyd oder Menthol/Isomenthol,
dadurch gekennzeichnet, daß man sie mit Hilfe der Extraktivdestillation in einer Fraktionierkolonne bei Drücken von 1 bar bis 1 mbar unter Zusatz eines polaren Hilfsstoffes mit einem Siedebereich von 50°C bis 310°C der Reihe Amine, Ether, Amide, Ketone, Alkohole, Nitrile, Heterocyclen, Sulfoxide, Sulfone durchführt, wobei die Auswahl des polaren Hilfsstoffes in einem Vorversuch mit Hilfe der Dampfraumanalyse erfolgt.

2. Verfahren gemäß Anspruch 1, dadurch gekennzeichnet, daß man zur Auswahl des polaren Hilfsstoffes das zu trennende Diastereomerengemisch zusammen mit dem auszuwählenden Hilfsstoff in ein thermostatisiertes Gefäß einfüllt und die Zusammensetzung der entstehenden Dampfphase durch Gaschromatographie analytisch bestimmt, wobei aus den Einwaagen der Flüssigkeit, vermindert um die entstandene Dampfmenge und der analytisch bestimmten Dampfkonzentration der Trennfaktor ermittelt wird.

3. Verfahren gemäß Anspruch 1, dadurch gekennzeichnet, daß man zur Trennung der diastereomeren cis/trans Permethrinsäuremethylester Tetramethylensulfon als Hilfsstoff einsetzt.

4. Verfahren gemäß Anspruch 1, dadurch gekennzeichnet, daß man zur Trennung der Diastereomere Menthol/Isomenthol, Bernsteinsäurediamid als Hilfsstoff einsetzt.

## Claims

1. Process for the resolution of diastereomers from the series comprising cis/trans permethric acid methyl ester (methyl 3-(2,2-dichloro-vinyl)-2,2-dimethyl-cyclopropanecarboxylate) or cis/trans permethric acid ethyl ester (ethyl 3-(2,2-dichloro-vinyl)-2,2-dimethyl-cyclopropanecarboxylate) or cis/trans permethric acid chloride (3-(2,2-dichloro-vinyl)-2,2-dimethyl-cyclopropane-carboxylic acid chloride) or methyl cis/trans 2,2-dimethyl-cyclopropane-3-carboxylate 1-aldehyde or menthol/isomenthol, characterized in that it is carried out with the aid of extractive distillation in a fractionating column under pressures of 1 bar to 1 mbar with the addition of a polar auxiliary having a boiling range from 50°C to 310°C from the series comprising amines, ethers, amides, ketones, alcohols, nitriles, heterocyclic compounds, sulphoxides and sulphones, the polar auxiliary being selected in a preliminary experiment with the aid of vapour analysis.

2. Process according to Claim 1, characterized in that for selecting the polar auxiliary the diastereomer mixture to be resolved is introduced into a thermostatically controlled vessel together with the auxiliary to be selected, and the composition of the resulting vapour phase is determined analytically by gas chromatography, the resolution factor being determined from the amounts of liquid weighed out, reduced by the resulting amount of vapour and the analytically determined vapour concentration.

3. Process according to Claim 1, characterized in that tetramethylene sulphone is employed as the auxiliary for resolution of the diastereomeric cis/trans permethric acid methyl esters.

4. Process according to Claim 1, characterized in that succinic acid diamide is employed as the auxiliary for resolution of the diastereomers menthol/isomenthol.

## Revendications

1. Procédés pour séparer des diastéréoisomères de la série ester méthylique d'acide de cis/trans perméthrine (ester méthylique d'acide 3-(2,2-dichlorovinyl)-2,2-diméthylcyclopropanecarboxylique) ou ester éthylique d'acide de cis/trans perméthrine (ester éthylique d'acide 3-(2,2-dichlorovinyl)-2,2-diméthylcyclopropanecarboxylique) ou chlorure d'acide de cis/trans perméthrine (chlorure d'acide 3-(2,2-dichlorovinyl)-2,2-diméthylcyclopropanecarboxylique) ou 1-aldéhyde d'ester méthylique d'acide cis/trans-2,2-diméthylcyclopropane-3-carboxylique ou menthol/isomenthol, caractérisés en ce qu'on les met en oeuvre à l'aide de la distillation extractive dans une colonne de fractionnement à des pressions de 1 bar à 1 mbar en utilisant une substance auxiliaire polaire ayant une plage d'ébullition de 50°C à 310°C, de la série des amines, des esters, des amides, des cétones, des alcools, des nitriles, des hétérocycles, des sulfoxydes, des sulfones, le choix de la substance auxiliaire polaire étant effectué dans un essai préliminaire par analyse de la phase vapeur.

2. Procédés suivant la revendication 1, caractérisés en ce que, pour choisir la substance auxiliaire polaire, on introduit le mélange de diastéréoisomères à séparer conjointement avec la substance auxiliaire à choisir dans un récipient thermostaté et on détermine par analyse par chromatographie en phase gazeuse la composition de la phase vapeur produite, et on établit le facteur de séparation à partir des prises d'essai de liquide, diminuées de la quantité de vapeur produite et de la concentration de vapeur déterminée par l'analyse.

3. Procédés suivant la revendication 1, caractérisés en ce qu'on utilise la tétraméthylènesulfone comme substance auxiliaire pour la séparation des esters méthyliques d'acide de cis/trans perméthrine diastéréoisomériques.

4. Procédés suivant la revendication 1, caractérisés en ce qu'on utilise le diamide d'acide succinique comme substance auxiliaire pour la séparation des diastéréoisomères menthol/isomenthol.

FIG. 1

FIG. 2